# EUROPEAN PATENT SPECIFICATION

(11) **EP 3 313 460 B1**
(45) Date of publication and mention of the grant of the patent: **10.11.2021**
(21) Application number: 16818575.9
(22) Date of filing: 28.06.2016
(51) Int. Cl.: A61L 15/32, C12P 21/00, D01D 5/00, D01F 4/00, D01H 1/30, D06M 101/14

(54) **FABRICS AND METHODS OF MAKING THEM FROM CULTURED CELLS**
GEWEBE UND VERFAHREN ZUR HERSTELLUNG DAVON AUS KULTIVIERTEN ZELLEN
TISSUS ET LEURS PROCÉDÉS DE FABRICATION À PARTIR DE CELLULES CULTIVÉES

(30) Priority: 29.06.2015 US 201562186253 P
(43) Date of publication of application: 02.05.2018
(73) Proprietor: Modern Meadow, Inc., Nutley, New Jersey 07110 (US)
(72) Inventor: MARGA, Francoise Suzanne, Nutley, New Jersey 07110 (US); DE LEEUW, Monique, Nutley, New Jersey 07110 (US); CASSINGHAM, Darryl M., Nutley, New Jersey 07110 (US); FORGACS, Gabor, Nutley, New Jersey 07110 (US)
(74) Representative: Maiwald Patent- und Rechtsanwaltsgesellschaft mbH
(86) International application number: PCT/US2016/039743
(87) International publication number: WO 2017/003999

(56) References cited:
- WO-A1-2011/051983
- WO-A1-2016/073453
- WO-A1-2016/073453
- GB-A- 723 214
- GB-A- 723 215
- GB-A- 992 585
- US-A- 4 089 333
- US-A- 4 089 333
- US-A- 4 294 241
- US-A- 4 404 033
- US-A1- 2013 255 003

## Description

### FIELD

Described herein are methods of forming textiles from cell-cultured proteins as well the resulting textiles. In particular, described herein are methods of forming fibers of collagen and textiles from the fibers of collagen, in which the collagen has originated from non-animal sources, such as yeast, bacteria, and plants, or from animal waste products. The textiles formed from the methods described herein may have unique characteristics arising from the fabrication techniques described herein, including ultra-structural uniformity (e.g., uniform tanning and/or cross-linking through the diameter of the fibers) and properties (e.g., strength, size, hydrothermal stability, etc.).

### BACKGROUND

Traditionally, textiles (e.g., fabrics) made of collagen arise from animal hide or skin, including leather and are used in a vast variety of applications, including furniture upholstery, clothing, shoes, luggage, handbag and accessories, and transportation vehicle applications. Currently, hides skins of animals are used as raw materials for natural leather. However, hides and skins from livestock pose environmental concerns because raising livestock requires ever increasing amounts of feed, pastureland, water, and fossil fuel. Livestock also produces significant pollution for the air and waterways, especially ruminants.

In addition, use of animal hides and skins to produce leather is objectionable to socially conscious individuals. The global leather industry utilizes a by-product of the meat industry, which slaughters more than a billion animals per year. Some of the leather comes from countries with no animal welfare laws or have laws that go largely or completely unenforced. Leather produced without killing animals would have tremendous fashion novelty and appeal.

Although synthetic leather was developed to address some of these concerns, it lacks the quality, durability, and prestige of natural leather. Thus far, scientifically sound and industrially feasible processes have not been developed to produce natural leather. Accordingly, there is a need for a solution to demands for alternative to leather produced from live animals.

Natural leather is typically a durable and flexible material created by the tanning of animal hide and skin, often cattle hide. Tanning is generally understood to be the process of treating the hides and skins of animals to produce leather. Tanning may be performed in any number of well-understood ways, including but not restricted to vegetable tanning (e.g., using tannin extracts from organic sources), chrome tanning (chromium salts including chromium sulfate), aldehyde tanning (using glutaraldehyde or oxazolidine compounds), syntans (synthetic tannins, using aromatic polymers). It is expected that new tanning agents will continue to be developed.

Natural leather is typically prepared in three main parts: preparatory stages (including the beamhouse operations), tanning, and crusting. Surface coating may also be included. The preparatory stages prepare the hide/skin for tanning, and unwanted raw skin components are removed. The preparatory stages may include: preservation, soaking (rehydrating), liming, unhairing, fleshing (removing subcutaneous material), splitting, re-liming, deliming (to remove de-hairing and liming chemicals), bating (enzymatic degradation treatment), degreasing, bleaching, and, if to be tanned with a metallic salt or aldehyde compound then pickling (through use of specific acids and salt), etc.

Tanning is performed to convert proteins in the hide/skin into a stable material that will not putrefy, possess hydrothermal stability, and be suitable for a wide variety of purposes as "leather". In case of chromium based tannin the pH of the skin/hide may be adjusted (e.g., lowered, e.g. to pH 2.0-2.4) to allow the penetration of the chromium into the fiber structure; subsequently the pH and temperature may be raised ("basification" to a slightly higher level, e.g., pH 3.8-4.2) in order to complete the tanning reaction.

Crusting typically refers to the post-tanning treatment that precedes the drying and staking of the leather. Examples of crusting techniques include: shaving, splitting, retanning, dyeing, fatliquoring, various means of drying (e.g. setting out, sammying, toggling, vacuum drying, paste drying, etc.), conditioning, buffing, staking, milling with the option then to apply various coatings and impregnations to the leather.

In practice, the process of converting animal hides and skins into leather may include sequential steps such as: unhairing, liming, deliming and bating, pickling, tanning, neutralizing, dyeing and fatliquoring, drying and finishing. The unhairing process may chemically remove the hair (e.g., using an auxiliary such as sodium sulphide in an alkali solution), while the liming step (e.g., using an alkali) may further complete the hair removal process and swell ("open up") the collagen to cause fiber bundle splitting and increase chemical reactivity through amino acid modification. During tanning, the skin structure is stabilized through the collagen reacting with complex ions of chromium. Depending on the compounds used, the color and texture of the leather may change. Tanned leather may be much more flexible than an untreated hide, and also more durable.

Skin, or animal hide, is formed primarily of collagen, a fibrous protein. Collagen is a generic term for a family of at least 28 distinct collagen types; animal skin is typically type I collagen (so the term collagen is typically assumed to be type I collagen), although other types of collagen may be used in forming leather. Collagens are characterized by a repeating triplet of amino acids, -(Gly-X-Y)*ₙ*-, so that approximately one-third of the amino acid residues are in collagen are glycine. X is often proline and Y is often hydroxyproline. Thus, the structure of collagen may consist of twined triple units of peptide chains of differing lengths. Different animals may produce different amino acid compositions of the collagen, which may result in different properties (and differences in the resulting leather). Collagen fiber monomers may be produced from alpha-chains of about 1050 amino acids long, so that the triple helix takes the form of a rod of about 300 nm long, with a diameter of 1.5 nm. In the production of extracellular matrix by fibroblast skin cells, triple helix monomers may be synthesized and the monomers may self-assemble into a fibrous form. These triple helices may be held together by salt links, hydrogen bonding, hydrophobic bonding, and covalent bonding. Triple helices organize into fibers and fibers into fiber bundles. Variations of the crosslinking or linking may provide strength to the material. Fibers may have a range of diameters. In addition to type I collagen, skin (hides) may include other types of collagens.

Previous attempts to make engineered leathers have proven unsuccessful or impractical. For example, EP1589098 describes a method of growing fibroblasts seeded onto three-dimensional bioactive scaffolds. The scaffolds may be made from collagen waste material from a tanning process ("split"), microparticles of pure collagen, particle of collagen waste material, or synthetic scaffolds (e.g., made of polymers such as HYAFF). The addition of the scaffold material complicates and increases the expense of their proposed process, and also affects the properties of the leather produced this way.

In addition, woven and non-woven synthetic materials formed of collagen and other proteins (particularly polymeric proteins) have been described and manufactured for biomedical (e.g., implant) purposes. Such materials are typically not suitable for use as a textile for garments, furniture and accessories (e.g., clothing, shoes, etc.), because they are not tanned, and lack the physical and chemical properties necessary, and have also proven prohibitively expensive to manufacture in sufficient size and quantity.

Described herein are engineered textiles that may have properties similar or superior to natural leathers, but may be processed in a very different, and in some ways simpler manner, and may address many of the problems of natural and other previously-described engineered leathers including those identified above.

GB 723 215 A relates to a process for the production of artificial filaments of globular water-insoluble proteins by wet-spinning of solutions of the proteins, comprising the step of treating the proteins, before dissolving them to prepare the spinning solution, with at least one fatty tanning substance consisting of a highly unsaturated triglyceride fatty oil and/or free fatty acid derived from such an oil. GB 723 214 A also relates to a process for the production of artificial filaments by the wet spinning of a solution of water-insoluble globular protein, characterized by the treatment of the protein prior to the spinning operation with a water-soluble tin salt in aqueous solution. US 4,089,333 A relates to the use of collagen in a method of treating a wound or burn which comprises dressing the surface of the wound or burn with a non-woven fabric essentially consisting of staple fibers of spun, regenerated collagen substantially free of telopeptides. GB 992 585 A relates to an open-fibred coherent collagenous material that comprises collagen fibres bound together by a fibrous derivative of gelatin, and to a method of making such a material. WO 2016/073453 A1 relates to reinforced engineered biomaterials, such as engineered leathers, and methods of making them.

### SUMMARY OF THE DISCLOSURE

This disclosure describes the use of proteins (natural or engineered) originating from a cell culture process to form fibers suitable for a wide range of textile manufacturing processes, including, but not restricted to those for non-woven, woven and knitted textiles (fabrics). In particular, described herein are fibers formed from cell-cultured materials that may be tanned as the fiber is formed, or in some variations tanned shortly after forming the woven, non-woven and knitted material.

Specifically, the present invention relates to a method of forming a textile, and a textile formed from cultured cells as claimed in the appended claims. In the present invention as claimed, the protein used is collagen.

In general, described herein are methods of forming protein fibers that may be formed into textiles and/or formed into an intermediate (e.g., yarn, thread, etc.) for later forming a textile. A textile (or cloth) may refer to a flexible material consisting of a network of fibers, yarn e.g., or thread. The textile formed may be primarily formed of the protein fiber, specifically the collagen fibers having the characteristics described herein, or they may be partially formed of these fibers (e.g., greater than 20%, greater than 25%, greater than 30%, greater than 35%, greater than 40%, greater than 45%, greater than 50%, greater than 55%, greater than 60%, greater than 65%, greater than 70%, greater than 75%, greater than 80%, greater than 85%, etc.). The fibers formed as described herein may be combined with other fibers, natural (e.g., cotton, silk, etc.) or synthetic (e.g., nylon, polyesters, etc.).

The methods described herein include forming fibers by forming a solution of collagen as protein (e.g., forming a solution in which the protein is a monomer, dimer, and/or small polymer), tanning the protein within the solution, which allows near-perfect penetration of the tanning agent into the resulting fibers and textile, then extruding fibers of the protein from the solution. These fibers may then be used to form a thread or yarn that is then used to form a textile (e.g., when making woven or knitted textiles) or used directly to make the textile (e.g., when making nonwoven textiles). Although according to the method as claimed the tanning is performed in the solution, which may be referred to as a polymer solution, a dope solution, or simply a dope, other methods described herein may also include tanning after extruding (or additional tanning after extruding) the fiber. Cross-linking may also be performed in the solution before, during, and/or after tanning; alternatively, cross-linking may be done after extruding the fibers. One or more additional treatments may be performed on the fibers, by including an agent in the solution. For example, a fire-retardant agent may be added to the solution so that it is integrated into the fibers. Compounds such as borates, polybenzimidazole (PBI), aramids, melamine, etc., may be included (e.g., in the solution) and integrated into the formed fibers to enhance the fire-retardant nature of the fibers and resulting textile. Alternatively or additionally, dyes, e.g., coloring agents, may also be included in the solution.

Described and claimed herein are methods of forming a textile that include: forming a solution of collagen as protein; tanning the protein within the solution; extruding fibers of the protein from solution; and forming the textile (or textile precursor) from the extruded fibers. Collagen is the protein that is used according to the invention as claimed to fabricate a textile or textile precursor material (yarn, thread, felt, etc.).

The method of forming a textile according to the invention thus includes: forming a solution of collagen; tanning the collagen within the solution; extruding fibers of the collagen from the solution; and forming the textile (or textile precursor) from the extruded fibers. The collagen source may in particular be a cruelty-free source, such as cultured cells (e.g., yeast, bacterial, etc.).

The method of forming a textile (or a textile precursor) according to the invention may include: harvesting collagen from collagen-secreting cells cultured *in vitro*; forming a solution of collagen protein; tanning and crosslinking the collagen within the solution; extruding fibers of the collagen from the solution; and forming the textile (or textile precursor) from the extruded fibers.

Any of the methods described herein may include extruding fibers within a particular size range and having a particular set of properties that may relate to the method of forming them. For example, extruding the fibers may comprise extruding fibers having a diameter of between 20-70 µm. Extruding the fibers may comprise extruding fibers having a uniform distribution of tanning through the diameter of the fibers (e.g., every fiber, >95% of the fibers, >90% of the fibers, >85% of the fibers, >80% of the fibers, >75% of the fibers, etc.), and/or be uniformly crosslinked through the entire diameter of the fiber (e.g., (e.g., every fiber, >95% of the fibers, >90% of the fibers, >85% of the fibers, >80% of the fibers, >75% of the fibers, etc.). For example, extruding the fibers may comprise extruding fibers comprising between 0.01% and 5% of tanning agent that is uniformly distributed through the entire diameter of the fiber.

In general, forming the solution may comprise solubilizing collagen within the solution. Forming the solution may comprise suspending collagen monomers in the solution, and/or a mixture of monomers, and short polymers (e.g., dimers, trimers, etc.). In general, the method may include forming the solution by suspending collagen (e.g., solubilizing collagen) in the solution to form a polymer solution.

Any of the methods described herein may use collagen arising from cultured sources. Thus, any of these methods may include harvesting collagen from collagen-secreting cells cultured *in vitro,* and forming the solution comprises including collagen from the harvested collagen in the solution.

As mentioned, the collagen may be cross-linked within the solution, e.g., by including a crosslinker in the solution (e.g., glutaraldehyde, (1-ethyl-3-(3-dimethylaminopropyl) carbodiimide hydrochloride or "EDC", etc.). Alternatively or additionally the fibers may be cross-linked after extruding from the solution. For example, crosslinking may comprise crosslinking with glutaraldehyde. As mentioned above, tanning the collagen within the solution may include mixing a tanning agent into the solution. Any appropriate tanning agent may be used; similarly any crosslinking agent may be used. For example, tanning agents and crosslinkers that can be used may include but are not limited to: chromium, aluminium, zirconium, titanium, iron, sodium aluminum silicate, formaldehyde, glutaraldehyde, oxazolidine, isocyanate, carbodiimide, polycarbamoyl sulfate, tetrakis hydroxyphosphonium sulfate, sodium p-[(4,6-dichloro-1,3,5-triazin-2-yl)amino] benzenesulphonate, vegetable tanning agents (both pyrogallol and catechol), syntans, etc.

In general, tanning may be for a very short time (relative to traditional tanning techniques for tanning, e.g., leather). For example the collagen solution may be exposed (may include) a tanning agent for less than 30 minutes (e.g., less than 25 minutes, less than 20 minutes, less than 15 minutes, less than 10 minutes, etc.) before extruding the fibers. For example, any of these methods may include tanning the collagen within the solution by adding a tanning agent into the solution for less than 20 minutes before extruding the fibers.

The fibers may be extruded to an appropriate shape (e.g., cross-sectional shape), including non-circular diameters, such as triangular, rectangular, square, trapezoidal, heptagonal, hexagonal, etc.

Extrusion may include any appropriate extrusion technique including spinning (e.g., wet, dry, dry jet-wet, melt, gel, and electrospinning, etc.), drawing, etc. For example, extruding fibers may comprise spinning the fibers.

As mentioned above, forming the textile may comprise forming a textile precursor, such as a yarn, from the fibers. In general, forming the textile may comprise knitting or weaving the fibers. Forming the textile may comprise forming a non-woven material from the fibers. Forming the textile may comprise combining the fibers with additional synthetic fibers, natural fibers, or natural and synthetic fibers.

The concentration of collagen within the solution to be extruded from may be controlled to allow controlled extrusion without breaking the fiber, or blocking/clogging the spinneret. For example, forming the solution may comprise forming a solution of collagen having a concentration of between 3 mg/ml and 30 mg/ml. For example, forming the solution may comprise forming a solution of collagen having a concentration of between 7 mg/ml and 15 mg/ml.

The rate of extrusion may be controlled, and may modify the resulting fibers (e.g., fiber length, etc.). For example, in any of these methods, the rate of extrusion may be between 70 µl/min and 300 µl/min of the solution.

Also described herein are fibers (specifically, collagen fibers), textile precursors (e.g., yarn, thread, etc.) made from these fibers, and textiles made from these fibers. The textiles according to the invention are formed using cultured cells, as described herein (in general, textiles may also be formed from any other source of the 'raw' protein). Described and claimed herein are textiles formed from cultured cells, the textile comprising a plurality of collagen fibers having a diameter of between 20-70 µm, wherein the collagen fibers comprise a concentration of tanning agent that is uniformly distributed through the diameter of the collagen fibers. For example, described herein are textiles formed from cultured cells comprising a plurality of collagen fibers having a diameter of between 20-70 µm, wherein the collagen fibers comprise between 0.01% and 30% tanning agent that is uniformly distributed through the diameter of the collagen fibers, further wherein the collagen fibers are uniformly cross-linked through the diameter of each fiber. The percentage of tanning agent within the collagen fibers may reflect the concentration of tanning agent used in the solution of collagen from which the fibers were formed (e.g., spun), as described herein. For example, the percentage of tanning agent within the collagen fibers may be between a lower value of 0.01%, 0.05%, 0.1%, 0.5%, 1%, 2%, 3%, 4%, 5%, 6%, 7%, 8%, 9%, 10%, etc., and an upper value of 30%, 27%, 25%, 22%, 20%, 19%, 18%, 17%, 16%, 15%, 14%, 13%, 12%, 11%, 10%, etc., where the lower value is always less than the upper value. As mentioned, the tanning agent within the collagen fibers is typically uniformly distributed, meaning that, for any of the collagen fibers, it has the same concentration profile that is reasonably uniform (e.g., within +/- 1%, 2%, 3%, 4%, 5%, etc.) of the same value through the entire cross-section of the fiber. This is in sharp contrast to traditional tanning methods, in which the concentration of tanning agent through a fiber may vary with distance from the outside of the fiber based on diffusion (particularly for larger, low penetration tanning agents, and when shorter tanning times (e.g., less than 12 hours) are used).

In general, as a result of the methods of forming the fibers (and textiles) described herein, the collagen fibers formed, and therefore the textile precursors and textiles formed, may be substantially uniform in size and/or properties. As used herein 'uniform' may mean having a variance of less than 20%, less than 15%, less than 10%, less than 12%, less than 10%, less than 8%, less than 5%, etc.

For example, the collagen fibers may be uniformly cross-linked through the diameter of each fiber. Additionally or alternatively, the collagen fibers may comprise between 0.01% and 5% of the tanning agent that is uniformly distributed through the diameter of the collagen fibers. The tanning agent within the collagen fibers may be any of the tanning agents described herein (e.g., chromium).

In general, the textiles formed as described herein may be formed into a sheet and/or roll. For example, the textile may be formed into a sheet having a surface area of greater than 100 cm² and/or a thickness of greater than 0.2 mm (e.g., greater than 0.3 mm, greater than 0.4 mm, greater than 0.5 mm, etc.). In one embodiment, the sheet may have a surface area of greater than 100 cm² and a thickness of greater than 0.03 mm. The collagen fibers may have a tenacity of between 1.5 and 2.5 g/denier. In some variations, the collagen fibers may have an elongation at break of between 25% and 40% (e.g., between 30-35%, etc.).

As mentioned above, the textile may include a mixture of collagen fibers and any other natural or synthetic fibers. For example, any of these textiles may include a plurality of synthetic or natural fibers mixed with the collagen fibers.

As mentioned above, the collagen fibers may have a non-circular diameter, which may alter the properties of the textile.

In general, the collagen fibers may be knitted or woven. In some variations, the textile comprises a non-woven textile.

The methods of forming fibers and textiles from these fibers described herein may offer numerous advantages. Many of these advantages may arise because of the surprising fact that tanning and/or crosslinking may be performed in the solution (e.g., the dope) prior to extruding fibers. For example, tanning and/or crosslinking in the solution results in a much faster tanning process. Typically in traditional tanning processes, depending on skin/hide thickness, the tanning period may necessarily have to be from 3 hours through one or more days. The methods described herein, in contrast are almost instantaneous. The methods described herein allow the tanning agent to penetrate the fibers easily by placing the tanning agent in-situ with the protein (specifically, the collagen) before they react. In the traditional leather industry the skin/hide must be chemically processed into a state of chemical dormancy through manipulation of temperature, pH (e.g., the iso-electric point) and other techniques to ensure penetration occurs before fixation. Otherwise the resulting material will only have a superficial or 'surface' tannage. Thus, the methods described herein may eliminate this need, as it may treat all fibers equally and individually.

The methods described herein may also allow the use of other tanning agents that are not typically used (though they may have beneficial properties) because of poor penetration when used with traditional leather formation methods. Failure to penetrate a skin/hide means that the resulting material will have inconsistencies. Such penetration problems are not an issue for the methods and fibers described herein.

As a result, the techniques described herein may also be an improvement in efficiency and environmental friendliness compared to other tanning techniques. With a real skin/hide tanning requires the completion of numerous operations that use a lot of water and can put a heavy load on the effluent system during pre-tanning, tanning, and at the end of tannage, as it is rare to exhaust all of the tanning materials (e.g., tanning agent) during the process. The methods described herein may allow one to use all of the tanning agents to achieve 100% exhaustion during fiber formation, and may require much less energy, water and effluent loading to make the fibers compared to regular leather.

In addition, the methods described herein may permit the incorporation of other materials and properties into the textiles formed using these methods. For example, the methods described herein may permit processing (e.g., by adding a material into the solution/dope) to modify or enhance flame retardance, water resistance, etc. In general, these methods may permit better individual treatment of each fiber. For example hydrophobic lubricating polymers may be incorporated. In another example, phosphonium-based tanning chemistry, which can yield fire retardance, may be easily used in the solution. As described herein, once a fiber (e.g., collagen) has been formed it may be formed already tanned, and it can immediately be individually treated with other technologies so that each and every fiber is equally treated and processed, thus giving ultra-high consistency, e.g. higher water resistance than can usually be achieved, as all of these fibers may be treated, whereas in traditional leather this cannot always be assured this, particularly on a thicker leather.

The methods described herein may also allow for fibers to be formed that can be of a specific geometric shape, as opposed to simply being round in cross-section. Such techniques are used in synthetic fibers and have been shown to have added performance, e.g., moisture transport, which has not yet been achieved in collagen and is made possible by the methods described herein.

Although the fibers described herein, and the resulting textiles formed therefrom are typically illustrated herein as formed of collagen arising from cell cultured (*in vitro*) sources, it should be understood that the collagen used in any of these methods and textiles are not limited to one particular type of collagen. In addition, synthetic (engineered, sequence-modified, etc.) collagens may be used in these methods and to form any of the fibers and textiles decribed herein. In addition, it should be understood that although the emphasis of this disclosure is on collagen sourced from *in vitro* (cell culture, e.g., yeast, bacteria, eukaryotic culture, etc.) sources, it may also be sourced from plant (including cultured plant cells or plant by-products, such as seeds, husks, hulls, etc.), and animal sources. For example, recycled leathers, waste leather, etc., may be used as a source of collagen.

### BRIEF DESCRIPTION OF THE DRAWINGS

FIG. 1 is a table (table 1) showing exemplary properties of textile fibers (these properties may be required for downstream processing in yarns and textile).
FIG. 2 schematically illustrates one variation of a method for forming a textile as described herein.
FIG. 3 schematically illustrates another variation of a method for forming a textile as described herein (e.g., a textile of collagen proteins).
FIG. 4 graphically illustrates the relationship between needle diameter and extrusion speed for an exemplary method of forming a collagen fiber as described herein (using a dope of 10 mg/ml collagen solution including a tanning agent and crosslinker).
FIG. 5 is a table comparing properties of fibers of collagen formed as described herein (e.g., as illustrated in FIG. 3) with traditional fibers of wool and silk.
FIG. 6 is a micrograph illustrating a silk fiber (on the left) having a diameter of approximately 10 mm with a collagen fiber formed as illustrated in FIG. 3, having a diameter of about 20 mm.
FIG. 7 is a graph comparing the effect of incorporating a crosslinker (in this example, glutaraldehyde) in the collagen solution (e.g., dope) prior to extruding, showing that there is no difference in the mechanical properties of the fiber when glutaraldehyde is used in either the maximum force (g) or the elongation (%) at break.

### DETAILED DESCRIPTION

Described herein are methods of producing polymeric protein fibers (using collagen as protein), textile precursors, and textiles from non-animal sources, including in particular cell culture sources. For example, described herein are methods of forming unwoven textiles, woven or knitted textiles and textile precursors (e.g., yarns, thread, etc.) from fibers composed of proteins, specifically of collagen, from humane sources including but, not restricted to, cell culture (for both prokaryotes or eukaryotes) of animals or plants, including yeast, bacteria, etc. The source of collagen, which is used as protein (e.g., protein monomers or small polymers) in the present invention may be natural or recombinant. Other proteins disclosed herein without being claimed are fibroin, sericin, casein, albumin. The proteins can be naturally produced by some mammalian cell type (e.g., fibroblasts, chondrocytes, smooth muscle cells, etc.), but can also be sourced from animals or plants. In other cases, the cells producing the protein (e.g., mammalian, yeast or bacterial, plant cells) can be engineered to form the protein of interest and either keep it within the cell or secrete into the extracellular space. In some variations, particularly when the protein is not secreted, the protein may be harvested from the cell (e.g., yeast or bacterium) by lysing. Alternatively or additionally, soluble proteins can be harvested directly from the medium. Also described herein are textiles made by these methods.

For example, described herein are methods of making collagen fibers for use in fabrication of textile fabrics. These textiles may have highly controlled leather-like characteristics that are superior to, or in some cases very different from, previously manufactured leathers.

In any of these variations, specialized, collagen-secreting animal cells (e.g., fibroblasts, smooth muscle cells, etc.) from animals such as bovine, porcine, ovine, etc., may be sourced from live animals by biopsy or extracted from animals slaughtered for their meat. Alternatively, existing cell-lines (mammalian cell lines) may be used, or bacterial and/or yeast cells expressing collagen or some other fiber-producing protein may be used. Cells may be grown under controlled conditions using standard cell culture techniques until sufficient number of them becomes available to produce a specific amount of protein (used to form the fabric).

In methods and systems in which adherent cells are used to produce collagen, cells may be plated on the surfaces of culture dishes with different curvature (both planer and curved, such as the inner surface of roller bottles; outer surface of a cylindrical cell culture mandrel) and cultured in the presence of ascorbic acid to secrete collagen. After several days (depending on cell type) a layer of collagen appears typically under the plated (and multiplied cells). Cells appear predominantly at the interface with the culture medium to maximize their exposure to nutrients. (A small number of cells remain in the secreted collagen matrix and at the lower interface with the culture vessel.) In addition secretion of collagen underneath of this cell layer is advantageous as the constrained space between the cells and the bottom of the culture vessel represents a constrained environment, which effectively acts as crowders. Collagen secreted into the culture medium above the cell layer tends to flow away from the cells making fibril and fiber formation inefficient. A significant concentration of soluble procollagen appears in the culture medium. The collagen layer is composed of substantially fibrils of variable length and diameter around 0.1 micron. In this example, decellularization of the construct may then be performed with known methods to remove the cell layer from the top of the collagen sheet, which is then reseeded with fresh cells. These on one had strongly attached to the collagen layer underneath and, on the other hand commence secrete their own collagen. Reseeding results in a thicker collagen sheet with most of the cells remaining again at the upper interface of the construct with the culture medium. This procedure can be continued until the desired thickness of the construct is achieved.

The engineered ECM can be digested into individual collagen fibrils or collagen monomers, by known methods, including enzymatic methods. These units can then be used to reconstitute the matrix by known methods to form collagen gels, which in turn can be extruded to form long fibers for spooling or stapled fibers for non-wovens or wovens or to produce yarns for knitting. In some variations, a solution of collagen from which fibers may be spun as described herein may include forming a solution of collagen by forming a solution of collagen monomers in the solution at an acidic pH.

The engineered ECM can be blended into small fragments and homogenized. The blended construct may then be dried and made into a powder-like substance through for example lyophilization. This powder can be conveniently stored until use. The powder can be rehydrated in a crosslinker-containing solution. In this process the viscosity of the resulting slurry and its mechanical properties can be conveniently controlled. The powder can also be mixed with other exogenous material for further control of material properties. The resulting slurry can be used for fiber formation by extrusion as described herein.

As used herein the term "collagen" may refer to a structural protein found in animal connective tissue. So far, 28 types of collagen have been identified and described. They can be divided into several groups according to the structure they form, for example, fibrillar (Type I, II, III, V, XI), facit (Type IX, XII, XIV), short chain (Type VIII, X), basement membrane (Type IV), and other types (Type VI, VII, XIII). Although collagen occurs in many places throughout the human body, over 90% of the collagen in the body, however, is type I. Collagen is a long, fibrous structural protein whose functions are quite different from those of globular proteins, such as enzymes. Collagens are major components of the extracellular matrix that supports most tissues and gives cells structure from the outside. Collagen has great tensile strength, and is the main component of fascia, cartilage, ligaments, tendons, bone and skin. Along with soft keratin, it is responsible for skin strength and elasticity, and its degradation leads to wrinkles that accompany aging. It strengthens blood vessels and plays a role in tissue development. It is present in the cornea and lens of the eye in crystalline form. As used herein, collagen may be of any appropriate type.

As used herein, the term "cell culture" may refer to the process by which cells are grown under controlled conditions, generally outside of their natural environment. In practice, the term "cell culture" may refer to the culturing of cells derived from multi-cellular eukaryotes, especially animal cells, as well as single-celled organisms, including bacteria and yeast. Cultured cells may therefore be derived from plants, fungi, microbes, including viruses, bacteria and protists. Cultures may be adherent (e.g., grown on a substrate) or suspended, or some combination of these. The methods described herein may use any appropriate cell culturing technique and may use any appropriate cell type.

The proteins for making fibers (including collagen used as protein according to the present invention) however may be sourced directly from live or dead animals or plants as well.

In some variations the methods described herein may use hydroentanglement as part of the method for forming nonwoven fabrics. As used herein, the term hydroentanglement may refer to a bonding process for wet or dry fibrous webs made by either carding, air-laying or wet-laying, the resulting bonded fabric being a nonwoven material. Hydroentanglement may use fine, high pressure jets of water which penetrate the web, hit the conveyor belt (or "wire" as in papermaking conveyor) and bounce back causing the fibers to entangle. Hydroentanglement is sometimes known as spun lacing, this term arising because the early nonwovens were entangled on conveyors with a patterned weave which gave the nonwovens a lacy appearance. It can also be regarded as a two-dimensional equivalent of spinning fibers into yarns prior to weaving. The water pressure has a direct bearing on the strength of the web, and very high pressures not only entangle but can also split fibers into micro- and nano-fibers, which give the resulting hydroentangled nonwoven a leather-like or even silky texture. This type of nonwoven can be as strong and tough as woven fabrics made from the same fibers.

Fibers formed from cell cultured materials as described herein may be formed by spinning, including wet spinning. Spinning may refer to a manufacturing process for creating polymer fibers that is a specialized form of extrusion that uses a spinneret to form multiple continuous filaments. There are many types of spinning: wet, dry, dry jet-wet, melt, gel, and electrospinning. In general a process for spinning may involve first converting the polymer being spun into a fluid state. This step may be unnecessary in some of the variations taught herein, in which cultured cells may already secrete and/or release the components of the fiber (e.g., collagen) into the culture medium. The fluid material including the polymer may then be forced through the spinneret, where it forms long fibers. Unlike spinning of traditional synthetic polymers, the spinning techniques described herein do not require (and may not use) heating/melting of the dope solution; instead, the collagen solution may be formed of monomeric collagen that is solubilized in an acidic solution. In general, the collagen solution and extruded collagen fibers may be maintained a low temperatures (e.g., less than 50°C, less than 45°C, less than 40°C, approximately room temperature, etc.), which may prevent degradation of the fibers. The extruded fibers may be solidified after extrusion. In some variations the fibers are extruded into a second solution that may modify the pH (e.g., neutralize the pH) of the fibers, and allowed to cool; it may cool to a rubbery state, and then a solidified state.

As used herein wet spinning may refer to the oldest of the five spinning processes. This process is used for polymers that need to be dissolved in a solvent to be spun. The spinneret is submerged in a chemical bath that causes the fiber to precipitate, and then solidify, as it emerges. The process gets its name from this "wet" bath. As will be described herein, this process may be modified to at least partially tan (e.g., expose to one or more tanning metals, such as chromium) the formed or forming fibers. A variant of wet spinning is dry jet-wet spinning, where the solution is extruded into air and drawn, and then submerged into a liquid bath.

Dry spinning may also be used for polymers that must be dissolved in solvent. It differs in that the solidification is achieved through evaporation of the solvent. This is usually achieved by a stream of air or inert gas. Because there is no precipitating liquid involved, the fiber does not need to be dried, and the solvent is more easily recovered. Extrusion spinning may use pellets or granules of the solid polymer that are fed into an extruder. The pellets are compressed, heated and melted by an extrusion screw, then fed to a spinning pump and into the spinneret. Direct spinning avoids the stage of solid polymer pellets, and instead the polymer melt is produced from the raw materials, and then from the polymer finisher directly pumped to the spinning mill. Gel spinning, also known as dry-wet spinning, may be used to obtain high strength or other special properties in the fibers. The polymer is in a "gel" state, only partially liquid, which keeps the polymer chains somewhat bound together. These bonds produce strong inter-chain forces in the fiber, which increase its tensile strength. The polymer chains within the fibers also have a large degree of orientation, which increases strength. The fibers are first air dried, then cooled further in a liquid bath. Some high strength polyethylene and aramid fibers are produced via this process. Electrospinning uses an electrical charge to draw very fine (typically on the micro or nano scale) fibers from a liquid, either a polymer solution or a polymer melt. Electrospinning shares characteristics of both electrospraying and conventional solution dry spinning of fibers. The process does not require the use of coagulation chemistry or high temperatures to produce solid threads from solution. This makes the process particularly suited to the production of fibers using large and complex molecules. Fibers may be drawn to increase strength and orientation. This may be done while the polymer is still solidifying or after it has completely cooled.

As used herein, a textile or cloth may refer to a flexible woven, non-woven or knitted material consisting of a network of natural or artificial fibers often referred to as thread or yarn. Yarn is produced by spinning raw fibers of wool, flax, cotton, or other material to produce long strands. Textiles may be formed by a variety of methods including but not restricted to weaving, knitting, crocheting, knotting, or felting. The words fabric and cloth may be used as synonyms for textile. However, in some contexts a textile may refer to any material made of interlacing fibers; a fabric may refer to any material made through weaving, knitting, spreading, crocheting, or bonding that may be used in production of further goods (garments, etc.). Cloth may be used synonymously with fabric. Felt is a textile that is typically produced by matting, condensing and pressing fibers together. As used herein a fiber may refer to a thread, filament or yarn from which a textile may be formed.

The methods described herein may be used to produce fabrics by unwoven fibers made from collagen as protein (secreted by cells or sourced by any other method). The collagen, as well as other proteins, like fibroin, sericin, casein, albumin, can be naturally produced by some mammalian cell types (fibroblasts, chondrocytes, smooth muscle cells, etc.). In other cases, cells (mammalian cells, yeast or bacteria) can be engineered to form the protein of interest and either keep it within the cell or secrete into the extracellular space. As mentioned above, to harvest the protein within the cell, the cells (e.g., yeast or bacteria) need to be lysed; soluble protein can be harvested directly from the medium.

### Example 1: Fiber formation from soluble proteins

Soluble proteins (specifically collagen as used in the present invention as claimed) can be isolated from a cell culture medium by techniques such as concentration by precipitation, dialysis and freeze-drying. For example, lyophilized material may be used to form the fibers by one of the 2 following methods. In method 1 (illustrated below), proteins may be solubilized in a buffer and the solution is deposited in thin layer on glass plate and air blown to induce the formation of fibers. After a crosslinking step, the fibers can be scraped from the glass. The fibers will be tanned with a modified process. In another method, method 2, protein (specifically collagen as used in the present invention as claimed) is dissolved into a solvent before wet spinning (extrusion), as briefly described above. To form fibers, the protein solution may be pushed through a nozzle directly into a coagulation bath. Shape and mechanical properties of the extruded fibers can be adjusted (e.g. by speed of extrusion, varying the concentration of the protein solution, etc.). As the filaments emerge from the spinneret into the wet spinning coagulation and cross-linking quench bath the filaments can be coalesced into a multifilament fiber and then spooled or collected into yarn. Alternatively, several multifilament fibers can be collected into a "tow bundle" which is then "piddled" or laid down uniformly into a box or container called a "tote". If the fibers were spooled they can first be drawn using a device such as a McCoy Ellison draw unit to further orient the fibrils in the filament thus increasing the strength (tenacity) of the filament while decreasing its diameter or denier. The drawn fibers can be rewound onto cylinders or can be wound directly onto a beam for use in weaving or warp knitting operations.

The first method (method 1) for forming collagen fibers may be similar to that described by Y. Wu, Kai Wang, Gisela Buschle-Diller, and Mark R. Liles ("Fiber Formation by Dehydration-Induced Aggregation of Albumin.") J. APPL. POLYM. SCI. 2013. Cells engineered to secrete protein (e.g., albumin) may be cultivated until the concentration of protein reaches a predetermined level (e.g., >1mg/ml). Proteins can be precipitated, e.g., by addition of ammonium sulfate (max 4M) or by cold ethanol (9:1 EtOH; medium (v/v). The precipitate may be recovered by centrifugation and the proteins (e.g., 10mg/ml) may be solubilized in a buffer (e.g., 10mM Na₂SO₄, 45mM DTT adjusted at pH 4.7 with HCl). The solution may be poured on a substrate such as a glass plate and dried by airflow at 30°C with low (e.g., less than 30%) humidity. The fibers may then be cross-linked (e.g., using formaldehyde vapor in methanol or EDC) and then rinsed with alcohol and air-dried. The fibers can also be detached by acetonitrile immersion or simply by scrapping them from the plate.

In any of the methods described herein, cultured cells may be used to grow the protein (specifically the collagen as used in the present invention as claimed) to produce the fibers, and these (collagen) fibers may be tanned as part of the fabrication process. Tanning may be performed in a manner that has not previously been possible or used when fabricating textiles. For example tanning of the fibers can be initiated either during their extrusion (e.g., in the solution/dope from which they are extruded) or after they are spooled. Tanning can also be performed on the yarn or the final fabric made of these fibers by textile making methods (see below). Tanning during (including before) extrusion allows essentially each protein filament to be tanned separately, and thus could give rise to filaments with vastly differing material properties depending on the specific tanning chemicals or their combination used.

In particular, tanning may occur by including a tanning agent (e.g., chromium, etc.) in the protein, specifically the collagen, solution prior to extruding the fiber; a cross-linker may also be included (either before, during, or after tanning using a tanning agent in the bath). For example, when forming (e.g., by extrusion) collagen fibers into a solution, the solution containing the filamentous proteins may also contain a tanning agent such as chromium (the ubiquitous tanning agent) and/or cross-linkers for example such as gluteraldehyde or EDC. The amount or concentration of such agents may be extremely low, as the fibers would be highly accessible. These tanned fibers may be further processed into a fabric with methods known in the textile industry to form woven, non-woven or knitted fabrics. Alternatively or additionally, tanning may be performed after the fibers have been turned into a yarn or fabric in which case it will mimic the tanning of animal hides.

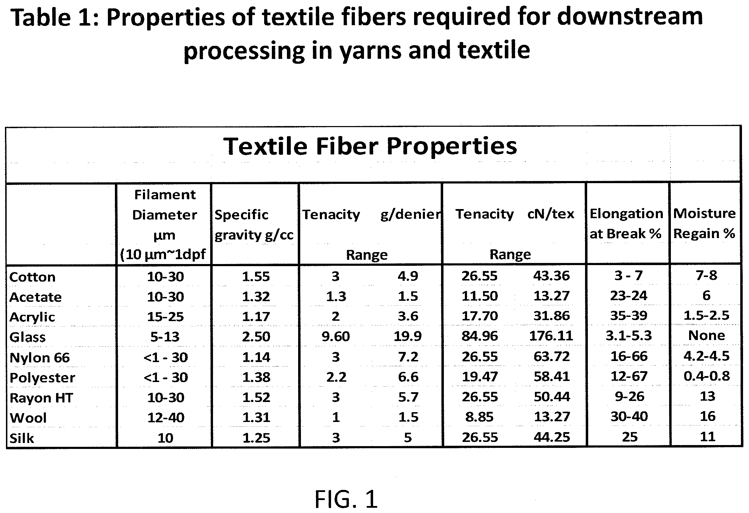

As mentioned above, any of these methods may be used to form non-woven textiles or fabrics. The fibers produced from soluble proteins as described herein may have physical properties similar to the properties of textile fibers (see, e.g., FIG. 1, showing Table 1, which describes properties of known textiles). These fibers formed of cell-cultured materials could, therefore, be processed into yarns and textile fabrics using downstream textile processing methods that are traditionally used for staple and filament fibers. Thus, the protein fibers, specifically the collagen fibers, can be used to manufacture non-woven, woven and knitted fabrics. They can also be used to manufacture both solid and hollow braided structures.

For example, if the fibers are collected as a tow, the tow can be crimped and then cut to a length of 1.8 to 2.5 cm to form "staple". The crimped staple fibers can then be baled for shipment to a yarn spinning facility or a non-woven processing facility. Staple yarns made from collagen can be used in a variety of non-woven manufacturing methods. To assure uniformity, several bales of protein staple fibers may be blended together. The fibers may then be "opened" to assure that there are no clumps of fibers and then the opened fibers are formed into a web. Any appropriate web forming process can be used, including, but not limited to, carding, aerodynamic or air laid, centrifugal dynamic and wet laid methods. Several layers of webs may be built up either by parallel or cross lapping of the webs. As mentioned above, the webs may be formed of fibers that have been tanned as described above. The webs may then be consolidated using mechanical, chemical, or thermal processes. Mechanical processes include, but are not limited to, stitchbonding, needlefelting or hydroentangling (spun lacing). Chemical methods of consolidating the web such as vinyl latex applied by saturation or printing can be used. If low melting thermoplastic staple fibers are blended with the collagen staple fibers then thermal web consolidation methods can be used such as calendering, radiant or convective heat or sonic bonding. Leather-like patterns can be embossed into the web. The consolidated web can take on the texture of the support screen or hydroentangling roll. If tanning has not been performed at the level of fibers then the consolidated web of protein fibers may be tanned and then dyed, printed and finished to achieve a wide variety of leather appearances.

Protein fibers can also be used to manufacture spun yarns and woven or knitted fabrics. Typical staple fiber processing methods in which the protein fibers can be used include, crimping, cutting to the desired length typically 1.8 to 2.5 cm, followed by blending, opening carding, combing, roving, forming a sliver, spinning into threads (yarns) twisting to increase strength and winding to make individual tubes or "packages" of single ends of yarn. Yarn spinning methods that can be used include, but are not limited to, ring spinning, open end spinning and air jet spinning. These tubes of yarn can be used directly in circular knitting and in manufacturing a filling or weft (cross or width direction) of a woven fabric.

Individual spools of protein yarn can be mounted in a creel containing many hundreds of such yarn packages. Each of the ends of protein yarn can be "sized" with a starch like chemical to temporarily stiffen it and then wound, each yarn positioned precisely on top of itself along the length of a long metal tube called a beam. The protein fibers on the beam can then be used to make the warp or length direction of a woven fabric.

The collagen yarns wound on a spool can be inserted across the width direction to make the filling or weft of a woven fabric. The collagen yarns are strong enough to withstand the five basic motions of weaving: let off, shedding, picking, beat up and fabric take-up. Types of looms on which the protein fibers can be woven, include but are not limited to, shuttle looms, projectile looms, rapier looms, water jet looms, air jet looms and jacquard looms.

The collagen yarns can be used to make any variety of woven fabrics including, but not limited to, plain weaves including warp rib, filling rib and basket weave. They can also be used to make all angles of twill weaves, satin weaves, dobby weaves, jacquard weaves, leno weaves, warp and filling pile weaves including corduroy, velveteen, velour and terry.

Protein fibers formed as described herein can be used to manufacture knitted fabrics. Protein fibers processed using the staple processing methods described above to form protein yarns can be used directly to manufacture weft knit fabrics. Weft knit fabric manufacturing methods using protein fiber based yarns include, but are not limited to single and double circular knit and flat bed or V bed knitting. The types of knitted stitches and fabrics that can be produced using protein fibers include, but are not limited to, eightlock, fashion knit, interlock, jersey, jersey flannel, links-links, Milanese, pile knit, velour, pique, rib knit, stockinette, suede and tuck stitch. Protein fibers can also be knit directly from sliver to avoid the necessity of spinning yarns.

Collagen filament fibers can be used in warp knitting. In warp knitting the collagen filament fibers are not spun into yarns but rather are used directly after drawing to increase strength and decrease denier. The first step of this process is to form a beam similar to the beams described above for use in woven fabrics. The types of warp knit fabrics that can be made from collagen filament fiber include tricot and raschel. Other types of knitted fabrics that can also be made include powernet (if blended with rubber or spandex), Simplex, double needle bar raschel for knitted velour and weft insertion.

All of the non-woven, woven and knitted fabrics made from collagen staple and filament fibers can be tanned and then dyed and finished using a variety of techniques appropriate for fine leather fabrics.

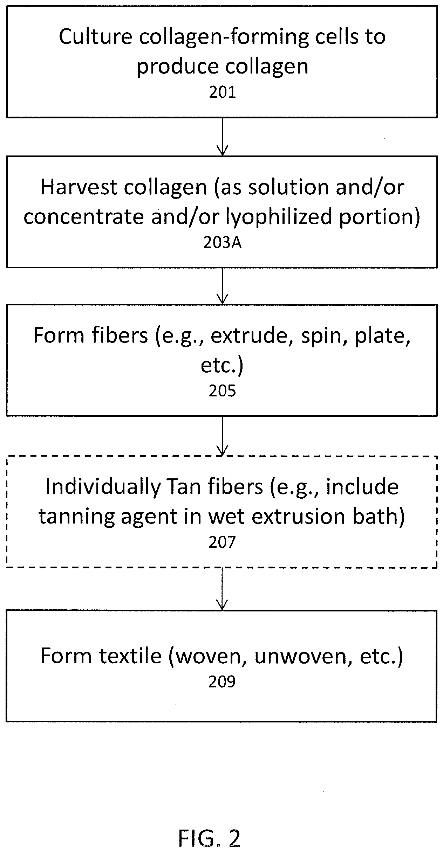

FIG. 2 illustrates a generic method of forming a textile (e.g., a leather-like textile) as described herein. In FIG. 2, the first step illustrated is culturing collagen-forming cells so that they produce collagen at a significant level 201. The level may be determined empirically (by direct or indirect sampling), including by sampling for the level of the protein or for the growth of the cells producing the collagen.

Thereafter, the collagen grown by the culturing of the cells may be harvested so that it is suspended in a solution (which may be concentrated and/or lyophilized) 203A. This solution including the collagen may then be used to form the fibers by any of the methods described herein or otherwise known (e.g., by extrusion, spinning, plate-lyophilization, etc.) 205. Optionally (as indicated by the dashed lines in FIG. 2), the fibers may be tanned, e.g., immediately after and/or during the fiber formation process 207 (for example, by adding and/or including a tanning agent in the solution prior to forming the fiber, e.g., by spinning). For example, a tanning agent such as chromium and/or other metals or fixative agent may be included in wet extrusion by either mixing it into the collagen solution or added into the coagulation bath. Thereafter a textile maybe formed 209 (e.g., woven, unwoven, etc.). The textile may be tanned as well or instead of tanning during/after fiber formation. An additional or alternative step of crosslinking may be included (not shown) one or more crosslinking agent in the solution of collagen from which the fibers are being formed and/or crosslinking after (e.g., immediately after) the fibers are formed.

Thus, in general, the protein fibers may be tanned during or immediately after formation, as fibers rather than hides and/or textiles. Although any appropriate method of forming the fibers may be used (including that shown, e.g., by Silver et al. in US 5,171,273), the materials and methods described herein are distinct as they provide for the first time a method (and resulting textile) that is tanned and/or crosslinked prior to forming the fibers that may then be used to form a textile (e.g., fabric). Surprisingly, the methods described herein may allow individual fibers to be formed pre- tanned (e.g., at a stage when the fibers and fiber components are easily individually accessible), and the resulting material may have properties not otherwise attainable in natural or synthetic materials (e.g., leathers) formed differently by the methods described herein and the resulting textile may have new properties. Such properties may not have been possible or observed with native leather before, including but not limited to super hydrophobicity, super oleophobicity, fire retardance, abrasion resistance, chromatographic color changing, thermal responsiveness, enhanced water resistance, etc. Generally, tanning of normal (animal harvested) leather is both difficult and requires a complex chemical understanding of the underlying process, which may be adjusted based on the individual properties of the hide being tanned. In contrast the methods described herein may, by tanning fibers when they are soluble or otherwise easily accessed, allow the complexity to be simplified and to achieve near-homogenous tanning by using lower concentrations and/or alternative types of tanning agents to stabilize the protein and prevent putrefaction. The methods described herein may allow optimal or near-optimal tanning by allowing access to every fiber of protein formed.

As mentioned above, the textiles formed as described herein may form a leather-like material, and these textiles may be used in any manner in which normal or synthetic leathers may be used. For example, the textiles described herein may be used, without limitation, as part of a garment, an accessory or a piece of furniture (e.g., shoes, bags, watchbands, jackets, pants, upholstery, etc.).

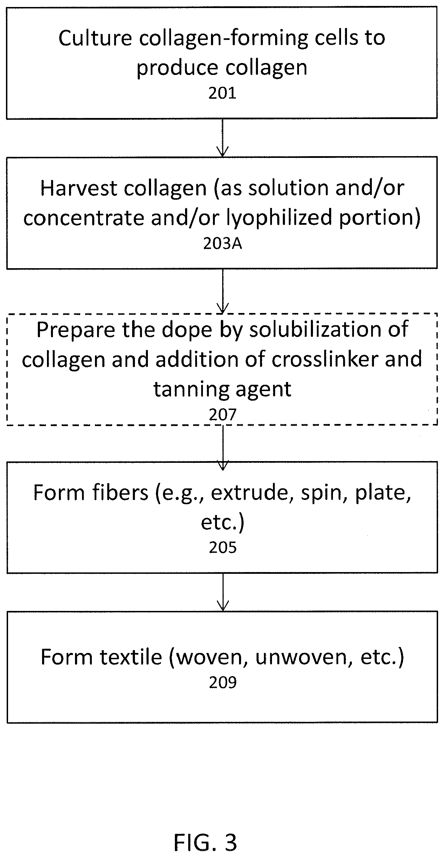

FIG. 3 illustrates another variation of a method for making a textile using the methods described herein. FIG. 3 is similar to FIG. 2, but includes preparing the solution (dope) 207 which incorporates a tanning agent and/or a crosslinker in the solution prior to forming the fiber (e.g., by extruding) 205.

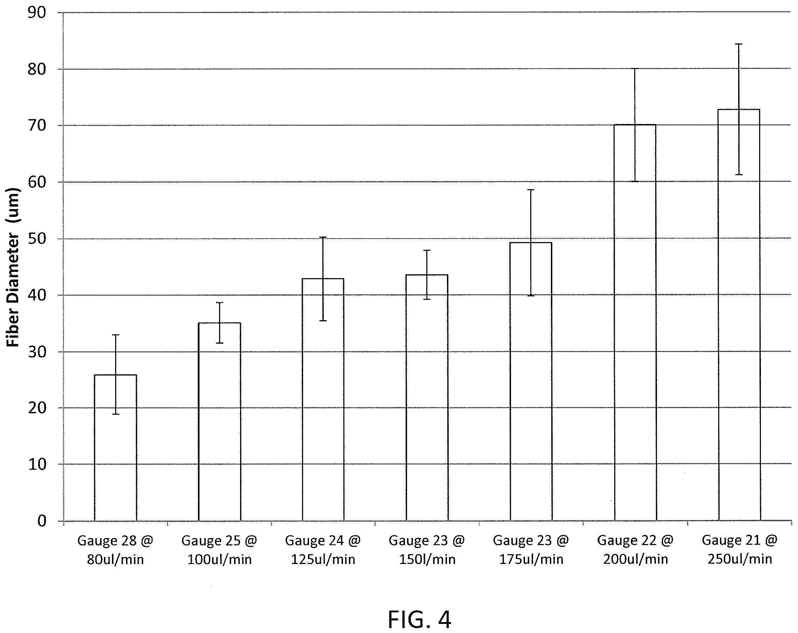

As mentioned above, any appropriate method of forming the fibers may be used. For example, when fibers may be extruded from a spinneret, which may include a needle (e.g., by spinning). FIG. 4 illustrates how needle diameter and the speed of extrusion of the protein solution (e.g., in this example collagen solution) into the bath affects the diameter of the fiber formed. In this example, the concentration of collagen in the collagen solution is approximately 10mg/ml.

In general, the inventors have found that, when using solution of collagen to form collagen fibers, the collagen concentration is an important parameter. A collagen solution of 10mg/ml was found to be optimal as forming fibers becomes more difficult as the concentration decreases. Although in some instances a low concentration of 3.4 mg/ml of neet collagen was successfully spun into a fiber using a gauge 25 needle, with the addition of crosslinker (glutaraldehyde) and a tanning agent (e.g., chromium), no fibers were able to form for collagen concentration at 3mg/ml, and a minimum of 7 mg/ml was necessary for fiber formation.

In addition, the length of the fiber formed may depend on the extrusion speed, the needle diameter, and/or the collagen concentration. If too little collagen is pushed through the needle, the fiber will break. If too much is pushed, the collagen will accumulate at the needle tip and no fiber is formed. For each needle gauge, the speed may be adjusted. As mentioned, the use (and type) of crosslinker and/or tanning agent may also modify the optimal speed, as the viscosity of the collagen solution may also increase. Surprisingly, despite the change in viscosity with the use of crosslinking and/or tanning agents in the solution, conditions were identified in which fibers could be formed (e.g., generally concentrations over 5 mg/ml at a rate of between 70 and 300 µl/min using a 21-28 gague needle).

The collagen fibers formed as described herein are remarkably uniform in properties, particularly in the distribution of tanning agent and/or crosslinker. A section through a diameter of a fiber shows that the distribution of tanning agent and/or crosslinking is nearly perfectly uniform in virtually all (e.g., >90%) of the collagen used in a textile formed as described herein.

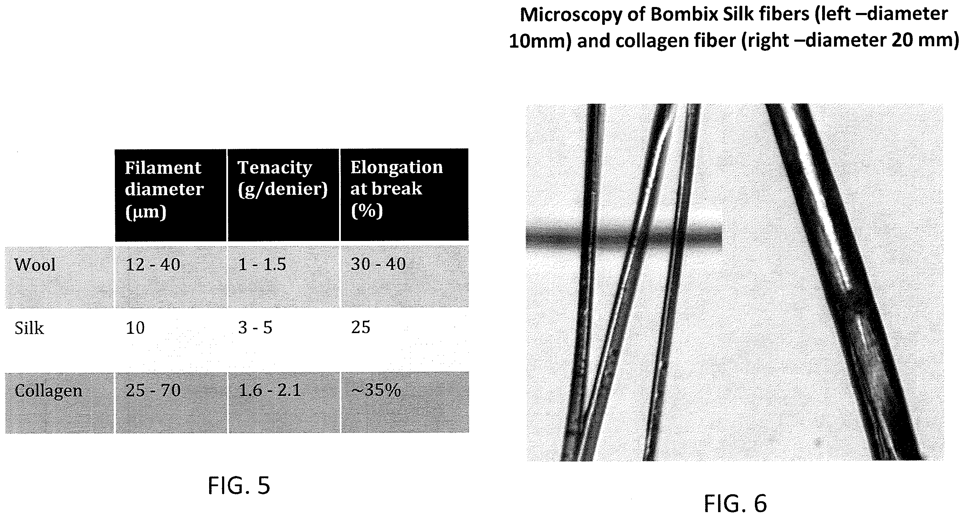

In addition, the collagen fibers formed as described herein (e.g., using a method such as that shown in FIG. 3) may have properties as good as or better than natural fibers or synthetic fibers, such as those shown in FIG. 1. FIG. 5 is a second table comparing physical properties (filament diameter, tenacity, elongation at break) of collagen fibers formed as described herein compared to natural wool and silk fibers. FIG. 6 also illustrates a microscopic comparison between Bombix Silk fibers (shown on the left, having a diameter of 10µm) and a collagen fiber formed as described herein (shown on the right, having a diameter of 20µm). These comparisons indicate that collagen fibers formed as described herein may have properties in the range required by the textile industry and may be useful to make fabrics that are more often fabricated using traditional textiles and methods.

### CROSSLINKERS AND TANNING AGENTS

As mentioned above, in general any appropriate cross-linking and tanning agent may be used. The addition of a crosslinker such as glutaraldehyde may be necessary to obtain a fiber that is water insoluble. Without crosslinking, collagen fibers may swell and re-solubilize in water. In variations in which cross-linking is done after extrusion (e.g., FIG. 2), the fibers may be placed overnight in a 2% solution (e.g., between about 1 to 4% solutions) of glutaraldehyde in acetone or methanol. In variations in which the crosslinker (crosslinking agent) is added before extrusion (e.g., in the collagen solution), the final concentration of glutaraldehyde may be 1.2% (although a similar range of 1 to 4% could be used).

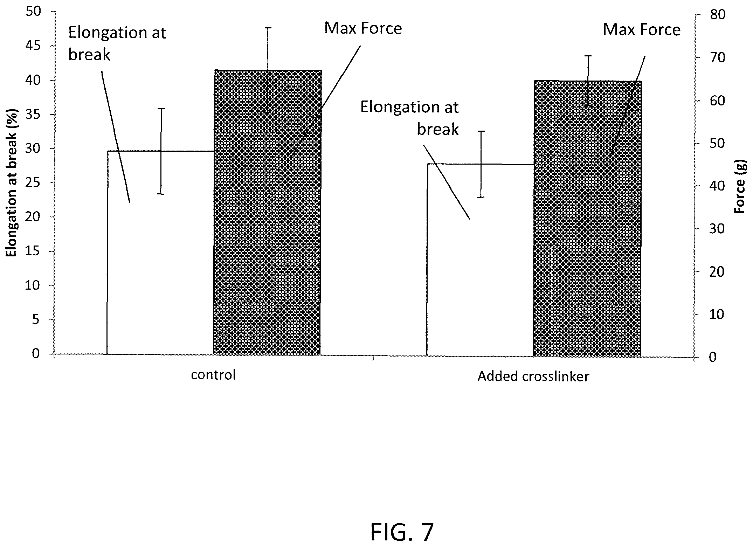

The addition of cross-linker during the methods of forming a fiber (and textile) as described herein (e.g., see FIG. 3) does not affect the mechanical properties of the fibers, as illustrated in FIG. 7. In FIG. 7, the maximal force (bars on right of each pair) and the elongation at break (bars on the left of each pair) are not significantly different for the fibers without and with addition of glutaraldehyde.

Similarly, tanning agents may be used to stabilize the collagen structure. One of the biggest benefits of tanning a fiber as described herein is the resulting hydrothermal stability of the fibers after tanning. In the traditional leather, the tanning agent has to penetrate in the hide, which may be difficult, time consuming and typically is non-uniform (as a gradient of concentration will inevitably result). Typically the complete hide is exposed to a particular (single) tanning agent, and no local treatment for a particular effect or property can be achieved. The methods described herein may allow tanning of every single fiber of the material. In addition to thermoresistance, other properties can be given to all or part of the fibers composing the fabrics. For example, part of the fibers can be fire resistant. A variety of fibers with different properties could be produce and incorporated into one material. Since the fibers are prepared separately, they could also be mixed with other textiles fibers to achieve other aesthetics or properties of the fabrics.

Testing of the fibers formed as described herein have shown that tanning (e.g., chromium tanning) of fibers for approximately 5 min in the solution prior to extruding the fiber results in fibers that have a similar hydrothermal property compared to native fibers and traditional leather. For example, fibers tanned and extruded as illustrated in FIG. 3 were placed in water and warmed to at least 95 °C without degradation, comparable to traditionally tanned leather materials.

### POST-EXTRUSION PROCESSING

Once fibers have been formed (e.g., extruded) as described herein, they may be used to form a pre-textile material (e.g., yarn, thread, etc.) and/or formed into the textile. These tanned fibers may also be further processed by one or more technique to modify the properties of the resulting textile. For traditional crusting steps (e.g., retanning, dyeing, fatliquoring, etc.) may be performed on the individual fibers and/or once the fibers have been formed into a textile.

For example, fatliquoring may include the addition of natural or synthetic lubricants to the fibers prior to forming them into a textile, which not only allow the fibers to dry without interfacial adhesion (sticking) but may also provide hydrophobicity, and other properties, to the fibers. This may provide advantages over regular fatliquoring, in that each individual fiber may be definitively treated with a set amount of lubricant, which is not guaranteed with natural leather due to the variability in internal fiber structure. These methods may also allow for alternate lubricants to be used that could not be normally considered due to issues with dispersion size, i.e., in natural leather for deep fiber penetration the emulsion size may be critical in order to be small enough to penetrate fully within the fiber matrix, but this may be alleviated by treating fibers individually as described herein. Further these methods may also provide improved tensile and tearing strength characteristics due to highly efficient fatliquoring. Finally the methods described herein may provide nearly complete (e.g., 100%) efficiency in exhaustion of the reagent, including the lubricant; in addition, the lubricant may be virtually immediately reacted with the fiber, as opposed to having to penetrate and then fix the lubricant as required by in natural leather. This results in an extremely energy efficient and material efficient process, using less chemicals, lower temperatures (as 'cold' fatliquoring can be employed due to not needing such a small dispersion size) and reduced water usage which consequently will reduce effluent requirements. In some variations, the percentage of fatliquoring (e.g., the percentage of oil lubricant by weight) may be less than 10% (e.g., between 0.1-15%, between 0.5-10%, etc.). In addition, one or more lubricants may be incorporated into the fiber itself during the formation process, including prior to extrusion, e.g., including a fatliquoring agent such as a lubricant within the protein solution prior. For example, a lubricant may be emulsified and included in the solution and/or added to the extruded fibers. In some variations the fatliquoring may be performed immediately after extruding the fibers, e.g., by extruding into a secondary solution that includes the fatliquoring agent. In some variations it may be preferably to include a fatliquoring agent only on the surface of the fibers (e.g., without substantially penetrating the fibers. Examples of fatliquoring agents may include: oils (e.g., sulfonated oils, mineral oil, etc.), fats (animal fats, vegetable fats, e.g., glycerides, etc.), synthetic lubricants, polysiloxanes, lubricating acrylic polymers, dry lubricants, etc. The fibers and/or a textile made from the fibers described herein may be made water resistant (e.g., "waterproofing") by the addition of an agent such as a hydrophobic agent, which can include, but are not limited to, hydrophobic lubricants (e.g. a modified polysiloxane such as Densodrin CD from BASF), fluorocarbons, hydrophobic acrylic polymers, chromium stearates, etc. The ability to make each fiber water resistant may also increase the consistency and varying levels of water resistance may be achievable in a controlled manner.

Retanning is performed with traditional leather materials to modify the qualities of the leather, including increasing/decreasing the concentration of the tanning agent, and/or modify the properties of the resulting fibers and/or textiles which may in term enhance further processing of the material, including dying. In any of the methods described herein retanning may be performed with the same or a different tanning agent. Retanning may be performed on the fibers (including on the pre-textile material) or on the textile. Retanning may be combined with any of the other post-extrusion steps described herein, including fatliquoring and/or dyeing. In addition, the order of these post-extrusion steps may be performed in any appropriate sequence (e.g., retanning then fatliquoring, etc.).

Dyeing adds color to the fibers and/or the resulting textile. Any appropriate dye may be used, particularly dyes that are appropriate for leather (e.g., collagen materials) although other dyes specifically designed for textiles may be used including those that have compatible reactivity, e.g. reactive dyes. As mentioned, in some variations a dye or dyes may be included in the protein (e.g., collagen) solution prior to extruding or immediately after extrusion. Dyes may include acid dyes (e.g., pre-metallized acid dyes), basic dyes, direct dyes, reactive dyes and sulfur dyes. A mordant dye (e.g., including a mordant to help with binding of the dye to the material) may also be used.

Other chemical treatments may also be added either in the protein (specifically the collagen) solution prior to extrusion, or after extrusion of filaments, such as: flame retardants, abrasion resistance treatments, thermos-regulating technologies, moisture management technologies, performance particulates, etc. The methods described herein may allow novel chemistries that have previously been difficult or unsuccessful when applied to traditional collagen materials such as natural leather, in attempts to impregnate into the fiber structure or otherwise be introduced to the fiber or textile. The methods described herein may successfully address the problem of applying such technologies that have in the past had to rely on a very rudimentary coating or lamination on to either or both of the surfaces of leather, which is often undesireable because such methods and systems can suffer from delamination, may badly affect the handling of the leather and ultimately take additional time, resources and equipment to apply such coatings/laminates.

## Claims

1. A method of forming a textile, the method comprising:
forming a solution of collagen as protein;
tanning the protein within the solution;
extruding fibers of the protein from the solution; and
forming the textile from the extruded fibers.

2. The method of claim 1, further comprising, prior to forming the textile, fatliquoring the fibers by addition of natural or synthetic lubricants, the percentage thereof being namely between 0,1 to 15%.

3. The method of claim 1, wherein the protein is obtained from harvesting collagen from collagen-secreting cells cultured *in vitro,* further wherein forming the solution comprises including collagen from the harvested collagen in the solution.

4. The method of claim 1, further comprising crosslinking the protein within the solution.

5. The method of claim 1, further comprising crosslinking the fiber after extruding from the solution.

6. The method of claim 4, wherein crosslinking comprises crosslinking with glutaraldehyde.

7. The method of claim 1, wherein extruding fibers comprises spinning the fibers.

8. The method of claim 1, wherein forming the textile comprises forming a yarn, knit, weave or a non-woven from the fibers.

9. The method of claim 1, wherein forming the textile comprises combining the fibers with additional synthetic fibers, natural fibers, or natural and synthetic fibers.

10. A textile formed from cultured cells, the textile comprising a plurality of collagen fibers having a diameter of between 20-70 µm, wherein the collagen fibers comprise a concentration of tanning agent that is uniformly distributed through the diameter of the collagen fibers.

11. The textile of claim 10, wherein the collagen fibers are uniformly cross-linked through the diameter of each fiber.

12. The textile of claim 10, wherein the tanning agent is chromium.

13. The textile of claim 10, wherein the collagen fibers have a tenacity of between 1,5 and 2,5 g/denier.

14. The textile of claim 10, further comprising a plurality of synthetic or natural fibers mixed with the collagen fibers.

15. The textile of claim 10, wherein the collagen fibers are knitted or woven.

16. The textile of claim 10, wherein the textile comprises a non-woven textile.

## Patentansprüche

1. Verfahren zum Herstellen einer Textilie, wobei das Verfahren umfasst:
Bilden einer Lösung von Kollagen als Protein;
Gerben des Proteins innerhalb der Lösung;
Extrudieren von Fasern des Proteins aus der Lösung; und
Bilden der Textilie aus den extrudierten Fasern.

2. Verfahren nach Anspruch 1, ferner umfassend, vor dem Bilden der Textilie, Fetten der Fasern durch Zugabe von natürlichen oder synthetischen Fettungsmitteln, deren Prozentsatz insbesondere zwischen 0,1 und 15% liegt.

3. Verfahren nach Anspruch 1, wobei das Protein durch ein Entnehmen von Kollagen von kollagenproduzierenden Zellen, die in vitro kultiviert werden, gewonnen wird, wobei ferner das Bilden der Lösung ein Aufnehmen von Kollagen von dem geernteten Kollagen in der Lösung umfasst.

4. Verfahren nach Anspruch 1, ferner umfassend Quervernetzen des Proteins innerhalb der Lösung.

5. Verfahren nach Anspruch 1, ferner umfassend Quervernetzen der Faser, nachdem sie aus der Lösung extrudiert wurde.

6. Verfahren nach Anspruch 4, wobei das Quervernetzen ein Quervernetzen mit Glutaraldehyd umfasst.

7. Verfahren nach Anspruch 1, wobei das Extrudieren von Fasern ein Spinnen der Fasern umfasst.

8. Verfahren nach Anspruch 1, wobei das Bilden der Textilie ein Herstellen eines Garns, eines Gewirkes, eines Gewebes oder eines Vlieses aus den Fasern umfasst.

9. Verfahren nach Anspruch 1, wobei das Bilden der Textilie ein Kombinieren der Fasern mit zusätzlichen synthetischen Fasern, natürlichen Fasern oder natürlichen und synthetischen Fasern umfasst.

10. Textilie, die aus kultivierten Zellen gebildet ist, wobei die Textilie mehrere Kollagenfasern mit einem Durchmesser von 20-70 µm umfasst, wobei die Kollagenfasern eine Konzentration eines Gerbstoffes umfassen, der gleichmäßig über den Durchmesser der Kollagenfasern verteilt ist.

11. Textilie nach Anspruch 10, wobei die Kollagenfasern gleichmäßig über den Durchmesser jeder Faser quervernetzt sind.

12. Textilie nach Anspruch 10, wobei der Gerbstoff Chrom ist.

13. Textilie nach Anspruch 10, wobei die Kollagenfasern eine Festigkeit zwischen 1,5 und 2,5 g/Denier aufweisen.

14. Textilie nach Anspruch 10, ferner umfassend mehrere synthetische oder natürliche Fasern, die mit den Kollagenfasern gemischt sind.

15. Textilie nach Anspruch 10, wobei die Kollagenfasern gewirkt oder gewebt sind.

16. Textilie nach Anspruch 10, wobei die Textilie eine Vliestextilie umfasst.

## Revendications

1. Procédé de formation d'un textile, le procédé comprenant de :
former une solution de collagène en tant que protéine ;
tanner la protéine dans la solution ;
extruder des fibres de la protéine à partir de la solution ; et
former le textile à partir des fibres extrudées.

2. Procédé selon la revendication 1, comprenant en outre, avant de former le textile, de nourrir en bain les fibres par l'addition de lubrifiants naturels ou synthétiques, le pourcentage de ceux-ci étant notamment compris entre 0,1 et 15 %.

3. Procédé selon la revendication 1, dans lequel la protéine est obtenue en récoltant du collagène à partir de cellules sécrétant du collagène cultivées in vitro, et dans lequel la formation de la solution comprend l'inclusion dans la solution de collagène provenant du collagène récolté.

4. Procédé selon la revendication 1, comprenant en outre la réticulation de la protéine dans la solution.

5. Procédé selon la revendication 1, comprenant en outre la réticulation de la fibre après son extrusion de la solution.

6. Procédé selon la revendication 4, dans lequel la réticulation comprend la réticulation avec du glutaraldéhyde.

7. Procédé selon la revendication 1, dans lequel l'extrusion des fibres comprend le filage des fibres.

8. Procédé selon la revendication 1, dans lequel la formation du textile comprend la formation d'un fil, d'un tricot, d'un tissage ou d'un non-tissé à partir des fibres.

9. Procédé selon la revendication 1, dans lequel la formation du textile comprend la combinaison des fibres avec des fibres synthétiques, des fibres naturelles, ou des fibres naturelles et synthétiques supplémentaires.

10. Textile formé à partir de cellules cultivées, le textile comprenant une pluralité de fibres de collagène ayant un diamètre compris entre 20 et 70 µm, dans lequel les fibres de collagène comprennent une concentration d'agent tannant qui est uniformément répartie sur le diamètre des fibres de collagène.

11. Textile selon la revendication 10, dans lequel les fibres de collagène sont réticulées uniformément à travers le diamètre de chaque fibre.

12. Textile selon la revendication 10, dans lequel l'agent tannant est du chrome.

13. Textile selon la revendication 10, dans lequel les fibres de collagène ont une ténacité comprise entre 1,5 et 2,5 g/denier.

14. Textile selon la revendication 10, comprenant en outre une pluralité de fibres synthétiques ou naturelles mélangées aux fibres de collagène.

15. Textile selon la revendication 10, dans lequel les fibres de collagène sont tricotées ou tissées.

16. Textile selon la revendication 10, dans lequel le textile comprend un textile non tissé.
